# EUROPÄISCHE PATENTSCHRIFT

(11) **EP 0 696 291 B1**
(45) Veröffentlichungstag und Bekanntmachung des Hinweises auf die Patenterteilung: **19.08.1998**
(21) Anmeldenummer: 94914314.3
(22) Anmeldetag: 28.04.1994
(51) Int. Cl.: C07F 9/6561, A61K 31/66, C07D 471/06, A61K 31/495

(54) **PYRIDO 1,2,3-DE]CHINOXALINDERIVATE, VERFAHREN ZU DEREN HERSTELLUNG UND IHRE VERWENDUNG IN ARZNEIMITTELN**
PYRIDO(1,2,3-DE)QUINOXALINE DERIVATIVES, PROCESS FOR PREPARING THE SAME AND THEIR USE IN MEDICAMENTS
DERIVES DE PYRIDO(1,2,3-DE)QUINOXALINE, LEUR PROCEDE DE PREPARATION ET LEUR UTILISATION DANS DES MEDICAMENTS

(30) Priorität: 28.04.1993 DE 4314593
(43) Veröffentlichungstag der Anmeldung: 14.02.1996
(73) Patentinhaber: SCHERING AKTIENGESELLSCHAFT, 13353 Berlin (DE)
(72) Erfinder: KRÜGER, Martin, D-13465 Berlin (DE); SEELEN, Werner, D-13593 Berlin (DE); TURSKI, Lechoslaw, D-13505 Berlin (DE)
(86) Internationale Anmeldenummer: DE9400494
(87) Internationale Veröffentlichungsnummer: WO9425472

(56) Entgegenhaltungen:
- WO-A-90/15058
- WO-A-93/08188
- JOURNAL OF ORGANIC CHEMISTRY Bd. 30, Nr. 8, 1965,, Seiten 2589 - 2593 RICHARDSON, A., JR. 'The Chemistry of 7-Aminoindoline and Certain Pyrrolo- and Pyrido(1,2,3-de)quinoxalines'

## Beschreibung

Die Erfindung betrifft Pyrido[1,2,3-de]chinoxalinderivate, deren Herstellung und Verwendung in Arzneimitteln.

Es ist bekannt, daß Chinoxalinderivate Affinität an die Quisqualat-Rezeptoren besitzen und sich auf Grund der Affinität als Arzneimittel zur Behandlung von Krankheiten des zentralen Nervensystems eignen. Ähnlich substituierte Chinoxalinderivate sind in WO 93/018188 beschrieben.

Es wurde nun gefunden, daß die erfindungsgemäßen Verbindungen geeignet sind, die Hyperaktivität excitatorischer Aminosäuren zu antagonisieren.

Die erfindungsgemäßen Verbindungen haben die Formel I 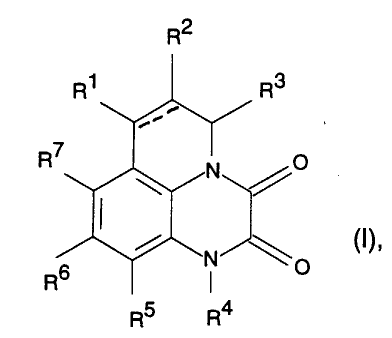 worin
R¹, R² und R³ jeweils Wasserstoff, -POXY oder Halogen und nicht gleichzeitig Wasserstoff sind,
R⁴ Wasserstoff oder OH,
R⁵, R⁶ und R⁷ gleich oder verschieden sind und Wasserstoff, Halogen, Nitro,
NR¹²R¹³, Cyano, CF₃, C₁₋₆-Alkyl oder C₁₋₄-Alkoxy bedeuten oder
R⁵ und R⁶ oder R⁶ und R⁷ einen ankondensierten Benzolring darstellen, und 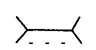 eine Einfach- oder Doppelbindung bedeutet,
wobei
X und Y gleich oder verschieden sind und Hydroxy, C₁₋₆-Alkoxy, -O-(CH₂)ₙ-O-, C₁₋ ₄-Alkyl oder NR⁹R¹⁰ bedeuten und n 1, 2 oder 3 ist und
R⁹ und R¹⁰, R¹² und R¹³ gleich oder verschieden sind und Wasserstoff, CO-C₁₋₆-Alkyl, Phenyl oder C₁₋₆-Alkyl , das gegebenenfalls mit C₁₋₄-Alkoxy oder einer gegebenenfalls mit C₁₋₄-Alkyl mono- oder disubstituierten Aminogruppe substituiert sein kann, oder gemeinsam mit dem Stickstoffatom einen gesättigten 5-7-gliedrigen Heterocyclus bilden, der ein weiteres Sauerstoff-, Schwefel- oder Stickstoffatom enthalten und gegebenenfalls substituiert sein kann oder einen ungesättigten 5-gliedrigen Heterocyclus bilden, der 1-3 N-Atome enthalten und substituiert sein kann, sowie deren Isomeren oder Salze.

Die Verbindungen der allgemeinen Formel I beinhalten auch die möglichen tautomeren Formen und umfassen alle möglichen Isomeren, sowie falls ein chirales Zentrum vorhanden ist, die Razemate oder Enantiomeren.

Unter Alkyl ist jeweils ein geradkettiger oder verzweigter Alkylrest zu verstehen wie beispielsweise Methyl, Ethyl, Propyl, Isopropyl, Butyl, Isobutyl, sek. Butyl, tert. Butyl, Pentyl, Isopentyl, Hexyl.

Unter Halogen ist jeweils Fluor, Chlor, Brom und Jod zu verstehen.

Bilden R⁹ , R¹⁰ und/ oder R¹², R¹³ gemeinsam mit dem Stickstoffatom einen gesättigten Heterocyclus, so ist beispielsweise Piperidin, Pyrrolidin, Morpholin, Thiomorpholin, Hexahydroazepin oder Piperazin gemeint, Der Heterocyclus kann 1-3-fach substituiert sein mit C₁₋₄-Alkyl oder Aryl wie Phenyl. Beispielsweise seien genannt N-Methyl-Piperazin, N-Phenylpiperazin, 2,6-Dimethyl-morpholin.

Bilden R⁹, R¹⁰ und R¹², R¹³ gemeinsam mit dem Stickstoffatom einen ungesättigten Heterocyclus, so seien beispielsweise Imidazol, Pyrazol, Pyrrol und Triazol genannt, die ein- bis zweifach mit Cyano, C₁₋₄-Alkyl, Phenyl oder CO₂C₁₋₆-Alkyl substituiert sein können.

Als Salze sind die physiologisch verträglichen Salze organischer und anorganischer Basen geeignet wie beispielsweise die gut löslichen Alkali- und Erdalkalisalze sowie N-Methylglukamin, Dimethyl-glukamin, Ethyl-glukamin, Lysin, 1,6-Hexadiamin, Ethanolamin, Glukosamin, Sarkosin, Serinol, Tris-hydroxy-methyl-amino-methan, Aminopropandiol, Sovak-Base, 1-Amino-2,3,4-butantriol und die physiologisch verträglichen Salze organischer und anorganischer Säuren wie HCl, HBr, H₂SO₄, Phosphorsäure, Zitronensäure, Weinsäure, Sulfonsäuren u.a.

Die Verbindungen der Formel I sowie deren physiologisch verträgliche Salze sind auf Grund ihrer Affinität zu den AMPA-Rezeptoren als Arzneimittel verwendbar. Auf Grund ihres Wirkprofils eignen sich die erfindungsgemäßen Verbindungen zur Behandlung von Krankheiten, die durch Hyperaktivität excitatorischer Aminosäuren wie zum Beispiel Glutamat oder Aspartat hervorgerufen werden. Da die neuen Verbindungen als Antagonisten excitatorischer Aminosäuren wirken und eine hohe spezifische Affinität zu den AMPA-Rezeptoren zeigen, indem sie den radioaktiv markierten spezifischen Agonisten (RS)-α-Amino-3-hydroxy-5-methyl-4-isoxazolpropionat (AMPA) von den AMPA-Rezeptoren verdrängen, eignen sie sich insbesondere zur Behandlung von solchen Krankheiten, die über die Rezeptoren excitatorischer Aminosäuren, insbesondere den AMPA-Rezeptor, beeinflußt werden.

Erfindungsgemäß können die Verbindungen zur Behandlung neurologischer und psychiatrischer Störungen verwendet werden, die durch die Überstimulation des AMPA-Rezeptors ausgelöst werden. Zu den neurologischen Erkrankungen, die funktionell und präventiv behandelt werden können, gehören zum Beispiel neurodegenerative Störungen wie Morbus Parkinson, Morbus Alzheimer, Chorea Huntington, Amyotrophe Lateralsklerose und Olivopontocerebelläre Degeneration. Erfindungsgemäß können die Verbindungen zur Prävention des postischämischen Zelluntergangs, des Zelluntergangs nach Hirntrauma, bei Schlaganfall, Hypoxie, Anoxie und Hypoglykämie und zur Behandlung der Senilen Demenz, Multiinfarkt Demenz sowie Epilepsie und Muskelspastik verwendet werden. Zu den psychiatrischen Erkrankungen gehören Angstzustände, Schizophrenie, Migräne, Schmerzzustände und Schlafstörungen sowie die Behandlung und Prävention der Entzugssymptomatik nach Drogenmißbrauch sowie bei Alkohol-, Kokain-, Benzodiazepin- oder Opiat-Entzug.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie zum Beispiel, Wasser, Gelantine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie drüber hinaus Hilfsstoffe wie Konservierungs-, Stabilisierungs-, Netzmittel oder Emulgatoren, Salze zur Veränderung des osmotischen Drucks oder Puffer.

Für die parenterale Anwendung sind insbesondere Injektionslösungen oder Suspensionen, insbesondere wäßrige Lösungen der aktiven Verbindungen in polyhydroxyethoxyliertem Rizinusöl, geeignet.

Als Trägersysteme können auch grenzflächenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder Kohlenwasserstoffträger oder -binder, wie zum Beispiel Lactose, Mais- oder Kartoffelstärke, geeignet. Die Anwendung kann auch in flüssiger Form erfolgen, wie zum Beispiel als Saft, dem gegebenenfalls ein Süßstoff beigefügt ist.

Die Dosierung der Wirkstoffe kann je nach Verabfolgungsweg, Alter und Gewicht des Patienten, Art und Schwere der zu behandelnden Erkrankung und ähnlichen Faktoren variieren. Die tägliche Dosis beträgt 0,5-1000 mg, vorzugsweise 50-200 mg, wobei die Dosis als einmal zu verabreichende Einzeldosis oder unterteilt in 2 oder mehreren Tagesdosen gegeben werden kann.

Die Herstellung der erfindungsgemäßen Verbindung erfolgt nach an sich bekannten Methoden. Beispielsweise gelangt man zu Verbindungen der Formel I dadurch, daß man
a) eine Verbindung der Formel II 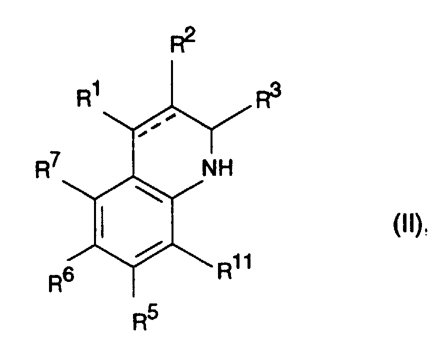 worin
   R¹¹ H, NO₂ oder -NH-R⁴ ist und R¹ bis R⁷ die obige Bedeutung haben, mit Oxalsäure oder reaktiven Oxalsäurederivaten umsetzt und gegebenenfalls nach Einführung und Reduktion der NO₂-Gruppe cyclisiert und, falls R¹, R² und/oder R³ Halogen bedeutet, gewünschtenfalls mit XYP-O-C₁₋₄-Alkyl umsetzt oder carbonyliert oder
b) eine Verbindung der Formel III 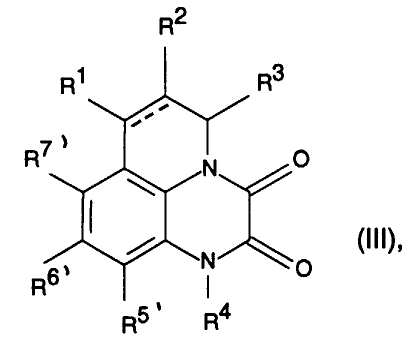 worin
   R¹ bis R⁴ die obige Bedeutung haben und R^{5'}, R^{6'} oder R^{7'} Wasserstoff oder eine elektronenziehende Gruppe bedeuten, halogeniert oder nitriert und gewünschtenfalls anschließend eine Estergruppe verseift oder eine Säuregruppe verestert oder amidiert oder eine Nitrogruppe reduziert zur Aminogruppe oder die Aminogruppe alkyliert oder acyliert oder eine Aminogruppe gegen Halogen oder Cyano austauscht oder eine Nitrogruppe oder Halogen einführt oder nucleophil substituiet oder die Isomeren trennt oder die Salze bildet.

Die Cyclisierung der Verbindungen der Formel II mit Oxalsäure oder einem reaktiven Oxalsäurederivat erfolgt einstufig oder auch zweistufig. Als bevorzugt ist das Zweistufenverfahren zu betrachten, bei dem das Diamin mit einem Oxalsäurederivat wie dem Oxalesterhalbchlorid oder reaktiven Oxalsäureimidazolidderivaten in polaren Lösungsmitteln wie cyclischen oder acyclischen Ethern oder halogenierten Kohlenwasserstoffen beispielsweise Tetrahydrofuran, Diethylether oder Methylenchlorid in Gegenwart einer Base wie organischen Aminen beispielsweise Triethylamin, Pyridin, Hünig-Base oder Diethylaminopyridin umgesetzt wird. Die anschließende Cyclisierung kann basisch oder auch sauer, vorzugsweise aber in saurem Milieu durchgeführt werden, wobei dem Lösungsmittel Alkohol zugesetzt werden kann.

Geeignete Basen für das Zweistufenverfahren stellen auch Alkalihydride dar wie NaH, die in inerten Lösungsmitteln wie Kohlenwasserstoffen oder Ethern eingesetzt werden.

Bedeutet R¹¹ in den Verbindungen der Formel II Wasserstoff, so kann nach der Acylierung mit dem reaktiven Oxalsäurederivat in üblicher Weise nitriert werden. Von den bekannten Nitrierungsmethoden ist die Umsetzung mit Natriumnitrat in Trifluoressigsäure oder mit Nitronium-tetrafluoroborat in chlorierten Kohlenwasserstoffen besonders geeignet. Die Reduktion der Nitrogruppe R¹¹ erfolgt in üblicher Weise katalytisch oder durch Reduktion mit Eisenpulver in Essigsäure bei erhöhter Temperatur oder auch mit Natriumsulfid und Ammoniumhydroxyd in Alkohol.

Die Einführung des Substituenten -POXY erfolgt nach gebräuchlichen Methoden, beispielsweise in Gegenwart einer Base wie organischen Aminen und eines Übergangsmetallkatalysators wie Tetrakis-(triphenylphosphin)-palladium(0) bei erhöhter Temperatur in polaren Lösungsmitteln wie Dimethylformamid oder cyclischen oder acyclischen Ethern oder halogenierten Kohlenwasserstoffen.

Die sich gegebenenfalls anschließende Verseifung einer Estergruppe kann basisch oder vorzugsweise sauer erfolgen, indem man bei erhöhter Temperatur bis zur Siedetemperatur des Reaktionsgemisches in Gegenwart von Säuren wie hoher konzentrierter wäßriger Salzsäure in Lösungsmitteln wie beispielsweise Trifluoressigsäure oder Alkoholen hydrolysiert. Phosphonsäureester werden bevorzugt durch Erhitzen in hochkonzentrierten wäßrigen Säuren wie zum Beispiel konzentrierter Salzsäure oder durch Behandlung mit Trimethylsilylhalogenid und anschließende Behandlung mit Wasser hydrolysiert.

Die Veresterung der Carbonsäure oder Phosphonsäure geschieht in an sich bekannter Weise mit dem entsprechenden Alkohol in Säure oder in Gegenwart eines aktivierten Säurederivats. Als aktivierte Säurederivate kommen zum Beispiel Säurechlorid, -imidazolid oder -anhydrid in Frage. Bei den Phosphonsäuren kann man die Veresterung durch Umsetzung mit Orthoestern gegebenenfalls unter Zusatz von Katalysatoren wie p-Toluolsulfonsäure erreichen.

Die Amidierung erfolgt an den freien Säuren oder an deren reaktiven Derivaten wie beispielsweise Säurechloriden, gemischten Anhydriden, Imidazoliden oder Aziden durch Umsetzung mit den entsprechenden Aminen bei Raumtemperatur.

Zur Einführung eines Halogen-Restes sind eine Vielzahl von bekannten Halogenierungsmethoden geeignet wie z.B. elektrophile aromatische Substitution. Beispielsweise gelingt die Jodierung mit Jod/Jodsäure in Eisessig nach einem Verfahren von Wirth et al. [Liebigs Ann. Chem. **634**, 84 (1960)] oder mit N-Jodsuccinimid.

Die Einführung einer NO₂-Gruppe gelingt durch eine Reihe von bekannten Nitrierungsmethoden. Beispielsweise kann mit Nitroniumtetrafluoroborat in inerten Lösungsmitteln wie halogenierten Kohlenwasserstoffen oder in Sulfolan oder Eisessig nitriert werden oder durch Nitriersäure in konzentrierter Schwefelsäure als Lösungsmittel bei Temperaturen zischen 0 °C und 30 °C.

Die Reduktion der Nitrogruppe zur Aminogruppe erfolgt katalytisch in polaren Lösungsmitteln bei Raumtemperatur oder erhöhter Temperatur unter Wasserstoffdruck. Als Katalysatoren sind Metalle wie Raney-Nickel oder Edelmetallkatalysatoren wie Palladium oder Platin gegebenenfalls in Gegenwart von Bariumsulfat oder auf Trägern geeignet. Statt Wasserstoff kann auch Ammoniumformiat in bekannter Weise benutzt werden. Reduktionsmittel wie Zinn-II-chlorid oder Titan-III-chlorid können ebenso verwendet werden wie komplexe Metallhydride eventuell in Gegenwart von Schwermetallsalzen. Es kann vorteilhaft sein vor der Reduktion die Estergruppe einzuführen. Nitrogruppen können auch selektiv in üblicher Weise mit Na₂S oder Natriumdithionit reduziert werden.

Wird eine Alkylierung einer Aminogruppe gewünscht, so kann nach üblichen Methoden beispielsweise mit Alkylhalogeniden oder nach der Mitsonubo Variante durch Umsetzung mit einem Alkohol in Gegenwart von Triphenylphosphin und Azodicarbonsäureester alkyliert werden oder man unterwirft das Amin einer reduktiven Aminierung mit Aldehyden oder Ketonen gegebenenfalls nacheinander mit zwei verschiedenen Carbonylverbindungen, wobei man gemischte Derivate erhält (Literatur z.B. Verardo et al. Synthesis 1993, 121; Synthesis 1991, 447; Kawaguchi, Synthesis 1985, 701; Micovic et al. Synthesis 1991, 1043).

Die Acylierung der Aminogruppe kann in üblicher Weise erfolgen, beispielsweise mit Säurehalogenid oder Säureanhydrid gegebenenfalls in Gegenwart einer Base wie Dimethylaminopyridin in Lösungsmitteln wie Methylenchlorid Tetrahydrofuran oder Pyridin oder nach der Scotten Baumann Reation.

Die Einführung der Cyanogruppe kann mit Hilfe der Sandmeyer-Reaktion erfolgen; beispielsweise kann man die aus den Aminoverbindungen mit Nitriten intermediär gebildeten Diazoniumsalze mit Alkalicyaniden in Gegenwart von Cu-I-cyanid umsetzen.

Die Einführung der Halogene Chlor, Brom oder Jod über die Aminogruppe kann beispielsweise auch nach Sandmeyer erfolgen, indem man die mit Nitriten intermediär gebildeten Diazoniumsalze mit Cu(I)chlorid oder Cu(I)bromid in Gegenwart der entsprechenden Säure (Salzsäure oder Bromwasserstoffsäure) oder mit Kaliumjodid umsetzt. Wenn statt eines Nitrits ein organischer Salpetrigsäureester benutzt wird, kann man die Halogene z.B. durch Zusatz von Methylenjodid oder Tetrabrommethan einführen.

Die Einführung von Fluor gelingt beispielsweise durch Balz Schremann-Reaktion des Diazoniumtetrafluorborates.

Die nucleophile Substitution wird nach literaturbekannten Methoden in Gegenwart einer Base durchgeführt und kann durch eine aktivierende elektronenziehende Gruppe wie z.B. Nitro, Cyano, Trifluormethyl vorzugsweise in ortho-Stellung begünstigt werden. Als Nucleophile sind beispielsweise primäre und sekundäre Amine, N-enthaltende gesättigte und ungesättigte Heterocyclen, Cyanide, Alkoholate u.a. geeignet. Die Umsetzung kann in polaren Lösungsmitteln wie Alkoholen, halogenierten Kohlenwasserstoffen, Dimethylsulfoxid, Dimethylacetamid, Acetonitril oder Wasser oder ohne Lösungsmittel vorgenommen werden. Als Basen sind anorganische Basen wie Alkali- oder Erdalkalihydroxide oder -carbonate oder organische Basen wie cyclische, alicyclische und aromatische Amine, wie DBU, Hünigbase, Pyridin oder Dimethylaminopyridin geeignet.
Als Base kann im Fall von Aminen das Nucleophil selbst im Überschuß benutzt werden, wobei man gegebenenfalls ohne weiteres Lösungsmittel arbeiten kann oder unter Druck.

Die Isomerengemische können nach üblichen Methoden wie beispielsweise Kristallisation, Chromatographie oder Salzbildung in die Enantiomeren bzw. E/Z-Isomeren aufgetrennt werden.

Die Herstellung der Salze erfolgt in üblicher Weise, indem man eine Lösung der Verbindung der Formel I mit der äquivalenten Menge oder einem Überschuß einer Alkali- oder Erdalkali-Verbindung, die gegebenenfalls in Lösung ist, versetzt und den Niederschlag abtrennt oder in üblicher Weise die Lösung aufarbeitet.

Soweit die Herstellung der Ausgangsverbindungen nicht beschrieben wird, sind diese bekannt oder analog zu bekannten Verbindungen, beispielsweise nach WO 93/08173 oder hier beschriebenen Verfahren herstellbar.

Die nachfolgenden Beispiele sollen das erfindungsgemäße Verfahren erläutern:

### Beispiel 1:

a) 1-(2,2,2-Trichlorethoxycarbonyl)-6-trifluormethyl-1,2-dihydrochinolin-2-phosphonsäurediethylester
   41,4 g 6-Trifluormethylchinolin werden bei 0 °C in 250 ml Acetonitril vorgelegt. 29 ml Chlorameisensäure-2,2,2-trichlorethylester werden zügig zugetropft und die Mischung 30 Minuten gerührt. 36,4 ml Triethylphosphit werden zugetropft und 49,8 g Natriumjodid portionsweise zugefügt. Man erwärmt 10 Minuten auf 50 °C, engt die Lösung ein, versetzt mit 400 ml Wasser und extrahiert viermal mit je 400 ml Essigester. Das Rohprodukt wird durch Säulenchromatografie gereinigt und aus Isopropylether umkristallisiert.
   Schmp.: 71 °C
b) 1-(2,2,2-Trichlorethoxycarbonyl)-6-trifluormethyl-1,2,3,4-tetrahydrochinolin-2-phosphonsäurediethylester
   71,6 g 1-(2,2,2-Trichlorethoxycarbonyl)-6-trifluormethyl-1 ,2-dihydrochinolin-2-phosphonsäuredi-ethylester werden in 800 ml Ethanol gelöst, mit 3,6 g Platin(IV)-oxid versetzt und bei Normaldruck hydriert. Es wird filtriert, eingeengt und aus Isopropylether umkristallisiert.
   Schmp.: 84° C
c) 6-Trifluormethyl-1,2,3,4-tetrahydrochinolin-2-phosphonsäurediethylester
   15,4 g 1-(2,2,2-Trichlorethoxycarbonyl)-6-trifluormethyl-1,2,3,4-tetrahydrochinolin-2-phosphonsäurediethylester werden in 400 ml Methanol gelöst, mit 7,2 g Zinkpulver versetzt und unter Stickstoff 1,5 Stunden bei 90 °C gerührt. Es wird über Kieselgur filtriert und das Filtrat eingeengt. Das Rohprodukt wird durch Säulenchromatografie gereinigt und aus Isopropylether umkristallisiert.
   Schmp.: 129 °C
d) 1-Ethoxyoxalyl-6-trifluormethyl-1,2,3,4-tetrahydrochinolin-2-phosphonsäurediethylester
   5,39 g 6-Trifluormethyl-1,2,3,4-tetrahydrochinolin-2-phosphonsäurediethylester werden in 100ml Tetrahydrofuran gelöst Unter Stickstoff und mit Eiskühlung werden 0,56g Natriumhydrid dazugegeben. Nach 30 Minuten bei +5 °C werden 2 ml Oxalsäureethylesterchlorid dazugetropft und die Mischung über Nacht bei Raumtemperatur gerührt. Es wird über Kieselgur filtriert und das Filtrat eingeengt. Das Rohprodukt wird durch Säulenchromatografie gereinigt und aus Isopropylether umkristallisiert.
   Schmp.: 115 °C
e) 1-Ethoxyoxalyl-8-nitro-6-trifluormethyl-1,2,3,4-tetrahydrochinolin-2-phosphonsäurediethylester
   5,25 g 1-Ethoxyoxalyl-6-trifluormethyl-1,2,3,4-tetrahydrochinolin-2-phosphonsäurediethylester werden in 100 ml Methylenchlorid gelöst und mit 4,78 g Nitroniumtetrafluoroborat versetzt. Es wird 23 Stunden bei Raumtemperatur gerührt. Die Lösung wird zweimal mit je 40 ml 5%iger Natriumhydrogencarbonat-Lösung gewaschen und eingeengt.
f) 9-Trifluormethyl-2,3-dioxo-1,2,3,5,6,7-hexahydro-pyrido[1,2,3-de]chinoxalin-5-phosphonsäurediethylester
   6,27 g 1-Ethoxyoxalyl-8-nitro-6-trifluormethyl-1,2,3,4-tetrahydrochinolin-2-phosphonsäurediethylester werden in 100 ml Eisessig gelöst und mit 13 g Eisenpulver versetzt. Die Mischung wird eine Stunde bei 90 °C gerührt. Nach dem Abkühlen auf Raumtemperatur wird abgesaugt, der Rückstand in 400 ml Essigester aufgenommen und die Suspension erneut abgesaugt. Das Filtrat wird mit 50 ml 5%iger Natriumhydrogencarbonat-Lösung gewaschen, über Magnesiumsulfat getrocknet und eingeengt. Das Rohprodukt wird durch Säulenchromatografie gereinigt und aus Essigester umkristallisiert. Man erhält 2,31 g 9-Trifluormethyl-2,3-dioxo-1,2,3,5,6,7-hexahydro-pyrido[1,2,3-de]chinoxalin-5-phosphonsäurediethylester vom Schmelzpunkt 161 °C.

### In analoger Weise wird hergestellt:

### 9-Chlor-2,3-dioxo-1,2,3,5,6,7-hexahydro-pyrido[1,2,3-de]chinoxalin-5-phosphonsäure-diethylester Schmp.: 173 °C

In grundsätzlich analoger Weise werden hergestellt:
9-Nitro-2,3-dioxo-1,2,3,5,6,7-hexahydro-pyrido[1,2,3-de]chinoxalin-5-phosphonsäurediethylester
9-Cyano-2,3-dioxo-1,2,3,5,6,7-hexahydro-pyrido[1,2,3-de]chinoxalin-5-phosphonsäuredimethylester

### Beispiel 2:

### 9-Trifluormethyl-2,3-dioxo-1,2,3,5,6,7-hexahydro-pyrido[1,2,3-de]chinoxalin-5-phosphonsäure

1,63 g 9-Trifluormethyl-2,3-dioxo-1,2,3,5,6,7-hexahydro-pyrido[1,2,3-de]chinoxalin-5-phosphonsäurediethylester werden in 40 ml Acetonitril unter Stickstoff bei Raumtemperatur vorgelegt. 3,63 ml Trimethylsilylbromid werden zügig zugetropft. Die Lösung wird 20 Stunden bei Raumtemperatur gerührt und eingeengt. Der Rückstand wird in 50 ml Wasser suspendiert, abgesaugt und mit Wasser gewaschen. Der Rückstand wird bei 105 °C und 0,7 Torr getrocknet. Man erhält 1,12 g 9-Trifluormethyl-2,3-dioxo-1,2,3,5,6,7-hexahydro-pyrido[1,2,3-de]chinoxalin-5-phosphonsäure vom Schmelzpunkt 262 °C.

In analoger Weise werden hergestellt.
9-Chlor-2,3-dioxo-1,2,3,5,6,7-hexahydro-pyrido[1,2,3-de]chinoxalin-5-phosphonsäure
   Schmp.: 305 °C
9-Nitro-2,3-dioxo-1,2,3,5,6,7-hexahydro-pyrido[1,2,3-de]chinoxalin-5-phosphonsäure
9-Cyano-2,3-dioxo-1,2,3,5,6,7-hexahydro-pyrido[1,2,3-de]chinoxalin-5-phosphonsäure 9-Trifluormethyl-2,3-dioxo-1,2,3,5,6,7-hexahydro-pyrido[1,2,3-de]chinoxalin-6-phosphonsäure
Schmp.: 294-295 °C

9-Chlor-2,3-dioxo-1,2,3,5,6,7-hexahydro-pyrido[1,2,3-de]chinoxalin-7-phosphonsäure
9-Trifluormethyl-2,3-dioxo-1,2,3,5,6,7-hexahydro-pyrido[1,2,3-de]chinoxalin-7-phosphonsäure
   Schmp.: ab 235 °C unter Zersetzung
8-Nitro-9-trifluormethyl-2,3-dioxo-1,2,3,5,6,7-hexahydro-pyrido[1,2,3-de]chinoxalin-5-phosphonsäure
   Schmp.: 318 °C unter Zersetzung
9-Chlor-8-nitro-2,3-dioxo-1,2,3,5,6,7-hexahydro-pyrido[1,2,3-de]chinoxalin-5-phosphonsäure
9-Trifluormethyl-8-piperidino-2,3-dioxo-1,2,3,5,6,7-hexahydro-pyrido[1,2,3-de]chinoxalin-5-phosphonsäure
8-Amino-9-trifluormethyl-2,3-dioxo-1,2,3,5,6,7-hexahydro-pyrido[1,2,3-de]chinoxalin-5-phosphonsäure
8-Fluor-9-trifluormethyl-2,3-dioxo-1,2,3,5,6,7-hexahydro-pyrido[1,2,3-de]chinoxalin-5-phosphonsäure
8-Morpholino-9-trifluormethyl-2,3-dioxo-1,2,3,5,6,7-hexahydro-pyrido[1,2,3-de]chinoxalin-5 phosphonsäure
2,3-Dioxo-1,2,3,5,6,7-hexahydro-benzo[h]pyrido[1,2,3-de]chinoxalin-5-phosphonsäure
5,6-Dioxo-1,2,3,5,6,7-hexahydro-benzo[g]pyrido[1,2,3-de]chinoxalin-3-phosphonsäure
8-[1(1,2,4-Triazolyl)]-9-trifluormethyl-2,3-dioxo-1,2,3,5,6,7-hexahydro-pyrido[1,2,3-de]-chinoxalin-5-phosphonsäure
8-(1-Pyrrolyl)-9-trifluormethyl-2,3-dioxo-1,2,3,5,6,7-hexahydro-pyrido[1,2,3-de]chinoxalin-5-phosphonsäure Schmelzpunkt >300 °C (Zersetzung)

### Beispiel 3:

a) 3-Brom-6-trifluormethyl-chinolin
   19,7 g 6-Trifluormethyl-chinolin werden in 200 ml Tetrachlormethan vorgelegt. 5,2 ml Brom werden zugetropft und die Lösung eine Stunde unter Rückfluß erhitzt. Innerhalb 20 Minuten werden 7,9 g Pyridin in 8 ml Tetrachlormethan zu der siedenden Lösung getropft. Nach einer Stunde läßt man abkühlen, dekantiert vom ausgefallenen Salz und engt die Lösung ein. Nach Säulenchromatografie erhält man 14,6 g 3-Brom-6-trifluormethylchinolin vom Schmelzpunkt 79 °C.
b) 6-Trifluormethyl-chinolin-3-phosphonsäurediethylester
   1,44 g Tetrakis-(triphenylphosphin)-palladium(0) in 16 ml Toluol, 3,8 ml Triethylamin und 3,6 ml Diethylphosphit werden bei Raumtemperatur unter Stickstoff vorgelegt und mit 6,90 g 3-Brom-6-trifluormethyl-chinolin versetzt. Es wird zwei Stunden bei 90 °C Badtemperatur gerührt. Die Mischung wird mit Ether verdünnt, abgesaugt und das Filtrat eingeengt. Nach Säulenchromatografie erhält man 8,3 g 6-Trifluormethyl-chinolin-3-phosphonsäurediethylester vom Schmelzpunkt 69 °C.
c) 6-Trifluormethyl-1,2,3,4-tetrahydrochinolin-3-phosphonsäurediethylester
   6,66 g 6-Trifluormethyl-chinolin-3-phosphonsäurediethylester werden in 70 ml Eisessig gelöst und über Platindioxid bei Raumtemperatur unter Normaldruck hydriert. Nach Säulenchromatografie erhält man 3,52 g 6-Trifluormethyl-1,2,3,4-tetrahydrochinolin-3-phosphonsäurediethylester vom Schmelzpunkt 112 °C.
d) 1-Ethoxyoxalyl-6-trifluormethyl-1,2,3,4-tetrahydrochinolin-3-phosphonsäurediethylester
   4,04 g 6-Trifluormethyl-1,2,3,4-tetrahydrochinolin-3-phosphonsäurediethylester werden in 80 ml Tetrahydrofuran unter Stickstoff und Eiskühlung vorgelegt. 0,43 g 80%iges Natriumhydrid werden unter Rühren zugegeben. Nach 30 Minuten werden 1,6 ml Oxalsäureethylesterchlorid dazugetropft. Man rührt zwei Stunden bei Raumtemperatur, filtriert über Kieselgur und engt ein. Nach Säulenchromatografie erhält man 4,87 g 1-Ethoxyoxalyl-6-trifluormethyl-1,2,3,4-tetrahydrochinolin-3-phosphonsäurediethylester als farbloses Öl.
e) 1-Ethoxyoxalyl-8-nitro-6-trifluormethyl-1,2,3,4-tetrahydrochinolin-3-phosphonsäurediethylester
   4,37 g 1-Ethoxyoxalyl-6-trifluormethyl-1,2,3,4-tetrahydrochinolin-3-phosphonsäurediethylester werden in 100 ml Dichlormethan vorgelegt und mit 3,98 g Nitroniumtetrafluoroborat versetzt. Es wird 24 Stunden bei Raumtemperatur gerührt, zweimal mit je 30 ml 5%iger Natriumbicarbonat-Lösung gewaschen und eingeengt. Nach Säulenchromatografie erhält man 4,25 g l-Ethoxyoxalyl-8-nitro-6-trifluormethyl-1,2,3,4-tetrahydrochinolin-3-phosphonsäurediethylester vom Schmelzpunkt 70-72 °C.
f) 9-Trifluormethyl-2,3-dioxo-1,2,3,5,6,7-hexahydro-pyrido[1,2,3-de]chinoxalin-6-phosphonsäurediethylester
   4,25 g 1-Ethoxyoxalyl-8-nitro-6-trifluormethyl-1,2,3,4-tetrahydrochinolin-3-phosphonsäurediethylester werden in 75 ml Eisessig gelöst und mit 8,5 g Eisenpulver versetzt. Es wird eine Stunde bei 90 °C Badtemperatur gerührt, abgesaugt und das Filtrat eingeengt. Nach Säulenchromatografie und Umkristallisation aus Essigester erhält man 1,45g 9-Trifluormethyl-2,3-dioxo-1,2,3,5,6,7-hexahydro-pyrido[1,2,3-de]chinoxalin-6-phosphonsäurediethylester vom Schmelzpunkt 253 °C.

### Beispiel 4:

a) 4-Hydroxy-6-trifluormethyl-chinolin
   Diese Verbindung wurde analog der im J. of the Am. Chem. Soc. **68**, 2685-2688 (1946) beschriebenen Verfahren aus 4-Trifluormethylanilin hergestellt.
b) 4-Brom-6-trifluormethyl-chinolin
   25,8 g Phosphoroxybromid werden bei Raumtemperatur vorgelegt und unter langsamem Erwärmen portionsweise mit 6,39 g 4-Hydroxy-6-trifluormethyl-chinolin versetzt. Es wird eine Stunde bei 90 °C gerührt, mit 120 ml Eiswasser zersetzt und mit 50 ml 32%iger Natronlauge alkalisch gestellt. Nach einer Stunde wird das ausgefallene Produkt abgesaugt, mit Wasser gewaschen und getrocknet. Die Kristalle werden in heißem Hexan gelöst, die unlöslichen Anteile abfiltriert und das Filtrat eingeengt. Man erhält 7,34g 4-Brom-6-trifluormethyl-chinolin vom Schmelzpunkt 56 °C.
c) 6-Trifluormethyl-chinolin-4-phosphonsäurediethylester
   1,73 g Tetrakis-(triphenylphosphin)-palladium(0) in 20 ml Toluol werden mit 4,6 ml Triethylamin, 4,3 ml Diethylphosphit und 8,28 g 4-Brom-6-trifluormethyl-chinolin versetzt und drei Stunden bei 90°C gerührt. Man fügt weitere 0,17g Tetrakis(triphenylphosphin)-palladium(0) und jeweils 0,4ml Triethylamin und Diethylphosphit hinzu und rührt eine Stunde bei 90 °C. Nach dem Abkühlen wird mit Ether verdünnt, abgesaugt, mit Ether gewaschen und das Filtrat eingeengt. Das Produkt wird durch Säulenchromatografie gereinigt. Man erhält 8,33 g 6-Trifluormethyl-chinolin-4-phosphonsäurediethylester als farbloses Öl.
d) 6-Trifluormethyl-1,2,3,4-tetrahydrochinolin-4-phosphonsäurediethylester
   8,32 g 6-Trifluormethyl-chinolin-4-phosphonsäurediethylester werden in 100 ml Eisessig gelöst, mit 1,0 g Platindioxid versetzt und bei Raumtemperatur hydriert. Nach Säulenchromatografie erhält man 5,44 g 6-Trifluormethyl-1,2,3,4-tetrahydrochinolin-4-phosphonsäurediethylester vom Schmelzpunkt 94 °C.
e) 1-Ethoxyoxalyl-6-trifluormethyl-1,2,3,4-tetrahydrochinolin-4-phosphonsäurediethylester
   5,05 g 6-Trifluormethyl-1,2,3,4-tetrahydrochinolin-4-phosphonsäurediethylester werden in 100 ml Tetrahydrofuran unter Stickstoff vorgelegt und mit 0,54 g 80%igem Natriumhydrid versetzt. Nach 30 Minuten werden 1,9 ml Oxalsäureethylesterchlorid bei Raumtemperatur zugetropft. Man rührt 15 Stunden bei Raumtemperatur, filtriert über Kieselgur und engt ein. Nach Säulenchromatografie erhält man 5,99 g 1-Ethoxyoxalyl-6-trifluormethyl-1,2,3,4-tetrahydrochinolin-4-phosphonsäurediethylester als farbloses Öl.
f) 1-Ethoxyoxalyl-8-nitro-6-trifluormethyl-1,2,3,4-tetrahydrochinolin-4-phosphonsäurediethylester
   5,68 g 1-Ethoxyoxalyl-6-trifluormethyl-1,2,3,4-tetrahydrochinolin-4-phosphonsäurediethylester werden in 100 ml Dichlormethan gelöst und mit 5,18 g Nitronium-tetrafluoroborat versetzt. Die Lösung wird 20 Stunden bei Raumtemperatur gerührt, mit 5%iger Natriumbicarbonat-Lösung gewaschen und eingeengt. Nach Säulenchromatografie erhält man 5,34 g 1-Ethoxyoxalyl-8-nitro-6-trifluormethyl-1,2,3,4-tetrahydrochinolin-4-phosphonsäurediethylester als farbloses Öl.
g) 9-Trifluormethyl-2,3-dioxo-1,2,3,5,6,7-hexahydro-pyrido[1,2,3-de]chinoxalin-7-phosphonsäurediethylester
   5,30 g 1-Ethoxyoxalyl-8-nitro-6-trifluormethyl-1,2,3,4-tetrahydrochinolin-4-phosphonsäurediethylester werden in 90 ml Eisessig vorgelegt, mit 10,6 g Eisenpulver versetzt und eine Stunde bei 90 °C gerührt. Die Suspension wird warm abgesaugt, mit Essigester nachgewaschen und das Filtrat eingeengt. Das Produkt wird durch Säulenchromatografie gereinigt und aus Essigester umkristallisiert. Man erhält 2,47 g 9-Trifluormethyl-2,3-dioxo1,2,3,5,6,7-hexahydro-pyrido[1,2,3-de]chinoxalin-7-phosphonsäurediethylester vom Schmelzpunkt 250 °C.

### Beispiel 5:

### 8-Nitro-9-trifluormethyl-2,3-dioxo-1,2,3,5,6,7-hexahydro-pyrido[1,2,3-de]chinoxalin-5-phosphonsäurediethylester

812 mg 9-Trifluormethyl-2,3-dioxo- 1,2,3,5,6,7-hexahydro-pyrido [1,2,3-de]chinoxalin-5-phosphonsäurediethylester werden in 15 ml Dichlormethan gelöst und bei Raumtemperatur mit 531 mg Nitronium-tetrafluoroborat versetzt. Es wird 15 Stunden gerührt und eingeengt. Der Rückstand wird in Essigester aufgenommen und mit 5%iger Natriumbicarbonat-Lösung gewaschen. Nach Säulenchromatografie erhält man 470 mg 8-Nitro-9-trifluormethyl-2,3-dioxo-1,2,3,5,6,7-hexahydro-pyrido[1,2,3-de]chinoxalin-5-phosphonsäurediethylester vom Schmelzpunkt 254 °C.

### Beispiel 6:

### 8-Amino-9-trifluormethyl-2,3-dioxo-1,2,3,5,6,7-hexahydro-pyrido[1,2,3-de]chinoxalin-5-phosphonsäurediethylester

226 mg 8-Nitro-9-trifluormethyl-2,3-dioxo-1,2,3,5,6,7-hexahydro-pyrido[1,2,3-de]chinoxalin-5-phosphonsäurediethylester werden in 20ml Ethanol gelöst, mit 50mg 10% Palladium auf Aktivkohle versetzt und bei Raumtemperatur und Normaldruck hydriert. Nach dem Absaugen des Katalysators wird eingeengt und aus Ethanol umkristallisiert. Man erhält 146 mg 8-Amino-9-trifluormethyl-2,3-dioxo-1,2,3,5,6,7-hexahydro-pyrido-[1,2,3-de]chinoxalin-5-phosphonsäurediethylester vom Schmelzpunkt 242 °C (unter Zersetzung).

### Beispiel 7:

### 8-(1-Pyrrolyl)-9-trifluormethyl-2,3-dioxo-1,2,3,5,6,7-hexahydro-pyrido[1,2,3-de]chinoxalin-5-phosphonsäurediethylester

211 mg 8-Amino-9-trifluormethyl-2,3-dioxo- 1,2,3,5,6,7-hexahydro-pyrido[1,2,3-de]chinoxalin-5-phosphonsäurediethylester und 66 mg 2,5-Dimethoxytetrahydrofuran werden eine Stunde bei 140 °C Badtemperatur umgesetzt. Nach Umkristallisation aus Ethanol erhält man 123 mg 8-(1-Pyrrolyl)-9-trifluormethyl-2,3-dioxo-1,2,3,5,6,7-hexahydro-pyrido [1,2,3-de]chinoxalin-5-phosphonsäurediethylester vom Schmelzpunkt 268 °C.

### Beispiel 8:

### 8-Jod-9-trifluormethyl-2,3-dioxo-1,2,3,5,6,7-hexahydro-pyrido[1,2,3-de]chinoxalin-5-phosphonsäure

406 mg 9-Trifluormethyl-2,3-dioxo-1,2,3,5,6,7-hexahydro-pyrido[1,2,3-de]chinoxalin-5-phosphonsäurediethylester in 2 ml Eisessig werden mit 0,05 ml Wasser, 0,025 ml konzentrierter Schwefelsäure, 178 mg Jod und 70 mg Jodsäure versetzt und 15 Stunden bei 80 °C Badtemperatur gerührt. Nach dem Einengen wird mit Wasser versetzt und das Produkt abgesaugt. Man erhält 60 mg 8-Jod-9-trifluormethyl-2,3-dioxo-1,2,3,5,6,7-hexahydro-pyrido[1,2,3-de]chinoxalin-5-phosphonsäure vom Schmelzpunkt 307 °C (unter Zersetzung).

### Beispiel 9

### 8-Jod-9-trifluormethyl-2,3-dioxo-1,2,3,5,6,7-hexahydro-pyrido[1,2,3-de]chinoxalin-5-phosphonsäurediethylester

100mg 8-Amino-9-trifluormethyl-2,3-dioxo- 1,2,3,5,6,7-hexahydro-pyrido[1,2,3-de]chinoxalin-5-phosphonsäurediethylester werden in 8ml ethanolischer Salzsäure aufgenommen und eingeengt und das Hydrochlorid in 6ml Dimethylformamid und 3ml Methylenjodid aufgenommen und bei 80°C Badtemperatur mit 0,08ml i-Amylnitrit versetzt. Nach 3h Rühren bei dieser Temperatur wird der Ansatz am Vakuum eingeengt. Man erhält 95mg 8-Jod-9-trifluormethyl-2,3-dioxo-1,2,3,5,6,7-hexahydro-pyrido[1,2,3-de]chinoxalin-5-phosphonsäurediethylester.

In grundsätzlich analoger Weise werden über Diazotierung im wässrigen Milieu hergestellt:
8-Fluor-9-trifluormethyl-2,3-dioxo-1,2,3,5,6,7-hexahydro-pyrido[1,2,3-de]chinoxalin-5-phosphonsäurediethylester

### Beispiel 10

### 8-(Piperidin-1-yl)-9-trifluormethyl-2,3-dioxo-1,2,3,5,6,7-hexahydro-pyrido[1,2,3-de]chinoxalin-5-phosphonsäurediethylester

Bei 0°C wird zu einer Aufschlemmung von 110mg 8-Amino-9-trifluormethyl-2,3-dioxo-1,2,3,5,6,7-hexahydro-pyrido[1,2,3-de]chinoxalin-5-phosphonsäurediethylester und 30mg Natiumborhydridtablettenstücken in 3 ml Tetrahydrofuran eine Lösung von 0,15ml einer 25%igen wässrigen Lösung von Glutardialdehyd und 0,45ml 3-M Schwefelsäure in 3ml Tetrahydrofuran:Methanol=2:3 getropft. Nach Abklingen der Reaktion werden nocheinmal 30mgNatiumborhydridtablettenstücken nachgeworfen und anschliessend 1h bei Raumtemperatur gerührt. Dann wird mit Natronlauge neutral gestellt und mit Essigester ausgeschüttelt. Die Essigesterphase wird getrocknet, filtriert und eingeengt. Nach Chromatographie über Kieselgel mit Toluol:Eisessig:Wasser=10:10:1 erhält man 50mg 8-(Piperidin-1-yl)-9-trifluormethyl-2,3-dioxo-1,2,3,5,6,7-hexahydro-pyrido[1,2,3-de]chinoxalin-5-phosphonsäurediethylester

In grundsätzlich analoger Weise werden hergestellt:
8-Morpholino-9-trifluormethyl-2,3-dioxo-1,2,3,5,6,7-hexahydropyrido[1,2,3-de]chinoxalin-5-phosphonsäurediethylester

### Beispiel 11

### 8-(1-Imidazolyl)-9-trifluormethyl-2,3-dioxo-1,2,3,5,6,7-hexahydropyrido[1,2,3-de]chinoxalin-5-phosphonsäure

150mg 8-Fluor-9-trifluormethyl-2,3-dioxo-1,2,3,5,6,7-hexahydro-pyrido[1,2,3-de]chinoxalin-5-phosphonsäurediethylester werden zusammen mit 600mg Imidazol 2 h bei 160°C Badtemperatur gerührt. Es wird dann die Reaktionsmischung in 10ml konzentrierter Salzsäure 1,5h am Rückfluss gekocht, eingeengt und in 5ml Wasser aufgenommen und abgesaugt. Man erhält 100mg 8-(1-Imidazolyl)-9-trifluormethyl-2,3-dioxo-1,2,3,5,6,7-hexahydro-pyrido[1,2,3-de]chinoxalin-5-phosphonsäure.

## Patentansprüche

1. Verbindungen der Formel I 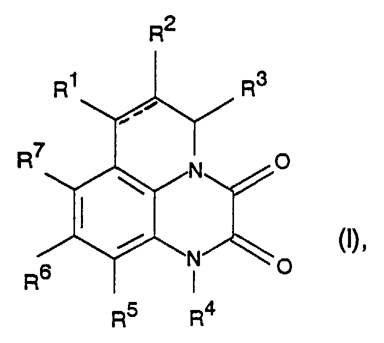 worin
R¹, R² und R³ jeweils Wasserstoff, -POXY, oder Halogen, und nicht gleichzeitig Wasserstoff sind,
R⁴ Wasserstoff oder OH,
R⁵, R⁶ und R⁷ gleich oder verschieden sind und Wasserstoff, Halogen, Nitro, NR¹²R¹³, Cyano, CF₃, C₁₋₆-Alkyl oder C₁₋₄-Alkoxy
bedeuten oder
R⁵ und R⁶ oder R⁶ und R⁷ einen ankondensierten Benzolring darstellen, und 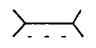 eine Einfach- oder Doppelbindung bedeutet,
wobei
X und Y gleich oder verschieden sind und Hydroxy, C₁₋₆-Alkoxy, -O-(CH₂)ₙ-O-, C₁₋₄-Alkyl oder NR⁹R¹⁰ bedeuten und n 1, 2 oder 3 ist und
R⁹ und R¹⁰, R¹² und R¹³ gleich oder verschieden sind und Wasserstoff, -CO-C₁₋₆-Alkyl, Phenyl oder C₁₋₆-Alkyl, das gegebenenfalls mit C₁₋₄-Alkoxy oder einer gegebenenfalls mit C₁₋₄-Alkyl mono- oder disubstituierten Aminogruppe substituiert sein kann oder gemeinsam mit dem Stickstoffatom einen gesättigten 5-7-gliedrigen Heterocyclus bilden, der ein weiteres Sauerstoff-, Schwefel- oder Stickstoffatom enthalten und gegebenenfalls substituiert sein kann,oder einen ungesättigten 5-gliedrigen Heterocyclus bilden, der 1-3 N-Atome enthalten und substituiert sein kann, sowie deren Isomeren oder Salze.

2. 8-(1-Imidazolyl)-9-trifluormethyl-2,3-dioxo-1,2,3,5,6,7-hexahydropyrido[1,2,3-de]chinoxalin-5-phosphonsäure
8-Jod-9-trifluormethyl-2,3-dioxo-1,2,3,5,6,7-hexahydro-pyrido[1,2,3-de]chinoxalin-5-phosphonsäure
8-(1-Pyrrolyl)-9-trifluormethyl-2,3-dioxo-1,2,3,5,6,7-hexahydro-pyrido[1,2,3-de]chinoxalin-5-phosphonsäure
8-Morpholino-9-trifluormethyl-2,3-dioxo-1,2,3,5,6,7-hexahydro-pyrido[1,2,3-de]chinoxalin-5-phosphonsäure
8-Amino-9-trifluormethyl-2,3-dioxo-1,2,3,5,6,7-hexahydro-pyrido[1,2,3-de]chinoxalin-5-phosphonsäure
9-Trifluormethyl-2,3-dioxo-1,2,3,5,6,7-hexahydro-pyrido[1,2,3-de]chinoxalin-6-phosphonsäure
9-Chlor-2,3-dioxo-1,2,3,5,6,7-hexahydro-pyrido[1,2,3-de]chinoxalin-5-phosphonsäure
9-Trifluormethyl-2,3-dioxo-1,2,3,5,6,7-hexahydro-pyrido[1,2,3-de]chinoxalin-5-phosphonsäure
9-Trifluormethyl-2,3-dioxo-1,2,3,5,6,7-hexahydro-pyrido[1,2,3-de]chinoxalin-7-phosphonsäure
9-Trifluormethyl-2,3-dioxo-1,2,3,5,6,7-hexahydro-pyrido[1,2,3-de]chinoxalin-5-phosphonsäurediethylester

3. Arzneimittel auf Basis der Verbindungen nach Anspruch 1 oder 2.

4. Verfahren zur Herstellung der Verbindungen der Formel I gemäß Anspruch 1, dadurch gekennzeichnet, daß man
a) eine Verbindung der Formel II 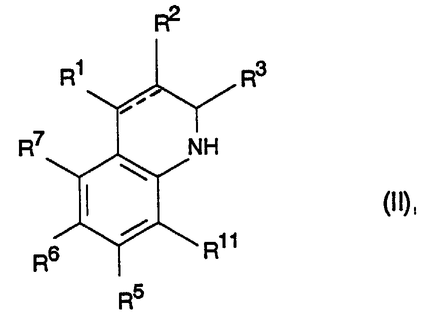 worin R¹¹ H, NO₂ oder -NH-R⁴ ist und R¹ bis R⁷ die obige Bedeutung haben, mit Oxalsäure oder reaktiven Oxalsäurederivaten umsetzt und gegebenenfalls nach Einführung und Reduktion der NO₂-Gruppe cyclisiert und, falls R¹, R² und/oder R³ Halogen bedeutet, gewünschtenfalls mit XYP-O-C₁₋₄-Alkyl umsetzt oder carbonyliert oder
b) eine Verbindung der Formel m 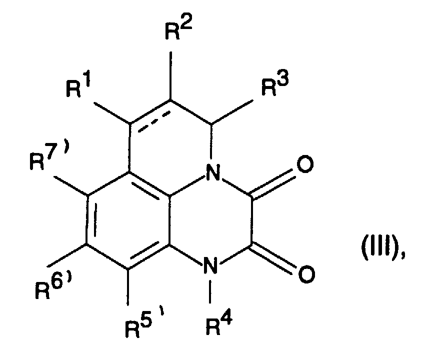 worin
R¹ bis R⁴ die obige Bedeutung haben und R^{5'}, R^{6'} oder R^{7'} Wasserstoff oder eine elektronenziehende Gruppe bedeuten, halogeniert oder nitriert und gewünschtenfalls anschließend eine Estergruppe verseift oder eine Säuregruppe verestert oder amidiert oder eine Nitrogruppe reduziert zur Aminogruppe oder eine Aminogruppe alkyliert oder acyliert oder eine Aminogruppe gegen Halogen oder Cyano austauscht oder eine Nitrogruppe oder Halogen einführt oder nucleophil substituiert oder die Isomeren trennt oder die Salze bildet.

## Claims

1. Compounds of formula I 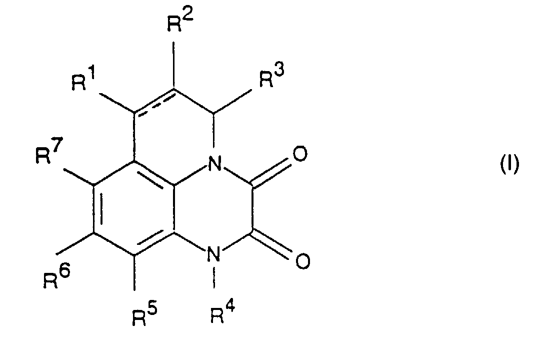 in which
R¹, R² and R³ are each hydrogen, -POXY or halogen and are not simultaneously hydrogen,
R⁴ is hydrogen or OH,
R⁵, R⁶ and R⁷ are the same or different and represent hydrogen, halogen, nitro, NR¹²R¹³, cyano, CF₃, C₁₋₆alkyl or C₁₋₄-alkoxy, or
R⁵ and R⁶ or R⁶ and R⁷ represent a fused benzene ring, and 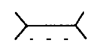 represents a single bond or a double bond,
wherein
X and Y are the same or different and represent hydroxy, C₁₋₆alkoxy, -O-(CH₂)ₙ-O-, C₁₋₄alkyl or NR⁹R¹⁰, and n is 1, 2 or 3, and
R⁹ and R¹⁰, R¹² and R¹³ are the same or different and represent hydrogen, -CO-C₁₋₆-alkyl, phenyl or C₁₋₆alkyl optionally substituted by C₁₋₄alkoxy or by an amino group that is optionally mono- or di-substituted by C₁₋₄alkyl, or together with the nitrogen atom form a saturated 5- to 7-membered heterocycle that may contain a further oxygen, sulphur or nitrogen atom and may optionally be substituted, or form an unsaturated 5-membered heterocycle that may contain from 1 to 3 nitrogen atoms and may be substituted, and their isomers or salts.

2. 8-(1-Imidazolyl)-9-trifluoromethyl-2,3-dioxo-1,2,3,5,6, 7-hexahydro-pyrido-[1,2,3-de]quinoxaline-5-phosphonic acid
8-iodo-9-trifluoromethyl-2,3-dioxo-1,2,3,5,6,7-hexahydro-pyrido[1,2,3-de]-quinoxaline-5-phosphonic acid
8-(1-pyrrolyl)-9-trifluoromethyl-2,3-dioxo-1,2,3,5,6,7-hexahydro-pyrido[1,2,3-de]-quinoxaline-5-phosphonic acid
8-morpholino-9-trifluoromethyl-2,3-dioxo-1,2,3,5,6,7-hexahydro-pyrido[1,2,3-de]-quinoxaline-5-phosphonic acid
8-amino-9-trifluoromethyl-2,3-dioxo-1 ,2,3,5,6,7-hexahydro-pyrido[1,2,3-de]-quinoxaline-5-phosphonic acid
9-trifluoromethyl-2,3-dioxo-1,2,3,5,6,7-hexahydro-pyrido[1,2,3-de]quinoxaline-6-phosphonic acid
9-chloro-2,3-dioxo-1,2,3,5,6,7-hexahydro-pyrido[1,2,3-de]quinoxaline-5-phosphonic acid
9-trifluoromethyl-2,3-dioxo-1,2,3,5,6,7-hexahydro-pyrido[1,2, 3-de]quinoxaline-5-phosphonic acid
9-trifluoromethyl-2,3-dioxo-1,2,3,5,6,7-hexahydro-pyrido[1,2,3-de]quinoxaline-7-phosphonic acid
9-trifluoromethyl-2, 3-dioxo-1,2,3,5,6,7-hexahydro-pyrido[1,2, 3-de]quinoxaline-5-phosphonic acid diethyl ester.

3. Medicament based on the compounds according to claim 1 or 2.

4. Process for the preparation of the compounds of formula I according to claim 1, characterised in that
a) a compound of formula II 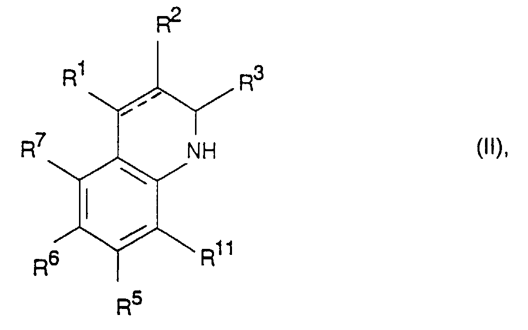 in which R¹¹ is H, NO₂ or -NH-R⁴ and R¹ to R⁷ are as defined above, is reacted with oxalic acid or a reactive oxalic acid derivative and optionally after introduction and reduction of the NO₂ group is cyclised, and, when R¹, R² and/or R³ is halogen, if desired the resulting compound is reacted with XYP-O-C₁₋₄alkyl or is carbonylated; or
b) a compound of formula III 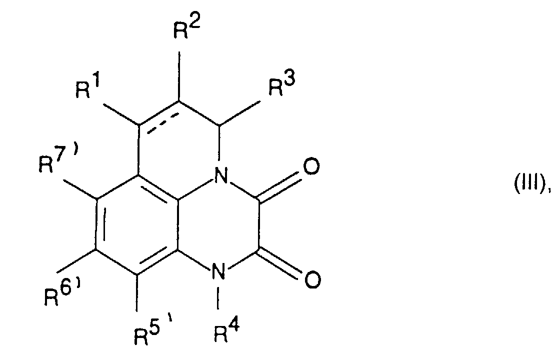 in which R¹ to R⁴ are as defined above and R^{5'}, R^{6'} and R^{7'} are hydrogen or an electron-attracting group, is halogenated or nitrated and, if desired, then an ester group is hydrolysed or an acid group is esterified or amidated or a nitro group is reduced to the amino group or an amino group is alkylated or acylated or an amino group is replaced by halogen or by cyano or a nitro group or halogen is introduced or nucleophilic substitution is carried out or the isomers are separated or the salts are formed.

## Revendications

1. Composés de formule I : 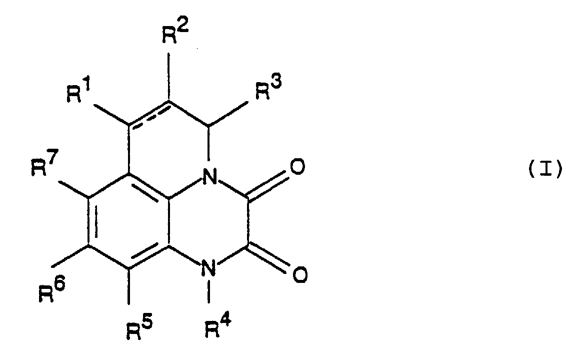 dans laquelle
R¹, R² et R³ représentent chacun un atome d'hydrogène, -POXY ou un atome d'halogène et ne sont pas simultanément l'hydrogène,
R⁴ représente un atome d'hydrogène ou un groupe OH,
R⁵, R⁶ et R⁷ sont identiques ou différents et représentent un atome d'hydrogène, un atome d'halogène, un groupe nitro, NR¹²R¹³, un groupe cyano, CF₃, un groupe alkyle en C₁-C₆ ou alcoxy en C₁-C₄, ou
R⁵ et R⁶ ou R⁶ et R⁷ représentent un cycle benzène condensé et 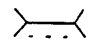 représente une simple ou double liaison,
X et Y étant identiques ou différents et représentent un groupe hydroxy, alcoxy en C₁-C₆, -O-(CH₂)ₙ-O-, alkyle en C₁-C₄ ou NR⁹R¹⁰ et n vaut 1, 2 ou 3, et
R⁹ et R¹⁰, R¹² et R¹³ sont identiques ou différents et représentent un atome d'hydrogène, -CO-(alkyle en C₁-C₆), phényle ou un groupe alkyle en C₁-C₆, lequel peut être éventuellement substitué par un groupe alcoxy en C₁-C₄ ou par un groupe amino éventuellement mono- ou disubstitué par un groupe alkyle en C₁-C₄ ou, conjointement avec l'atome d'azote, forment un hétérocycle saturé à 5 à 7 chaînons, qui peut contenir un autre atome d'oxygène, de soufre ou d'azote et peut être éventuellement substitué, ou forment un hétérocycle insaturé à 5 chaînons qui peut contenir 1 à 3 atomes d'azote et peut être substitué, ainsi que leurs isomères ou sels.

2. Acide 8-(1-imidazolyl)-9-trifluorométhyl-2,3-dioxo-1,2,3,5,6,7-hexahydro-pyrido[1,2,3-de]quinoxalino-5-phosphonique
Acide 8-iodo-9-trifluorométhyl-2,3-dioxo-1,2,3,5,6,7-hexahydro-pyrido[1,2,3-de]-quinoxalino-5-phosphonique
Acide 8-(1-pyrrolyl)-9-trifluorométhyl-2,3-dioxo-1,2,3,5,6,7-hexahydro-pyrido[l,2,3-de]quinoxalino-5-phosphonique
Acide 8-morpholino-9-trifluorométhyl-2,3-dioxo-1,2,3,5,6,7-hexahydro-pyrido[1,2,3-de]quinoxalino-5-phosphonique
Acide 8-amino-9-trifluorométhyl-2,3-dioxo-1,2,3,5,6,7-hexahydropyrido[1,2,3-de]quinoxalino-5-phosphonique
Acide 9-trifluorométhyl-2,3-dioxo-1,2,3,5,6,7-hexahydro-pyrido[1,2,3-de]quinoxalino-6-phosphonique
Acide 9-chloro-2,3-dioxo-1,2,3,5,6,7-hexahydro-pyrido[1,2,3-de]quinoxalino-5-phosphonique
Acide 9-trifluorométhyl-2,3-dioxo-1,2,3,5,6,7-hexahydro-pyrido[1,2,3-de]quinoxalino-5-phosphonique
Acide 9-trifluorométhyl-2,3-dioxo-1,2,3,5,6,7-hexahydro-pyrido[1,2,3-de]quinoxalino-7-phosphonique
9-Trifluorométhyl-2,3-dioxo-1,2,3,5,6,7-hexahydropyrido[1,2,3-de]quinoxalino-6-phosphonate de diéthyle

3. Médicaments à base des composés selon la revendication 1 ou 2.

4. Procédé pour la préparation de composés de formule I selon la revendication 1, caractérisé en ce que l'on fait réagir :
a) un composé de formule II : 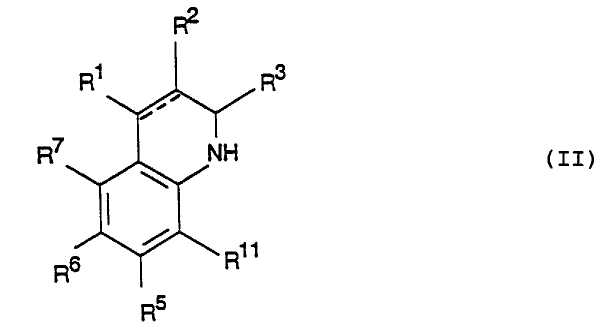 dans laquelle
R¹¹ représente H, NO₂ ou -NH-R⁴ et R¹ à R⁷ ont les significations données ci-dessus, avec l'acide oxalique ou des dérivés réactifs d'acide oxalique et éventuellement après introduction et réduction du groupe NO₂ on effectue une cyclisation et dans le cas où R¹, R² et/ou R³ représentent un atome d'halogène, si on le souhaite, on fait réagir avec XYP-O-(alkyle en C₁-C₄) ou carbonyler, ou
b) on effectue une halogénation ou une nitration du composé de formule III : 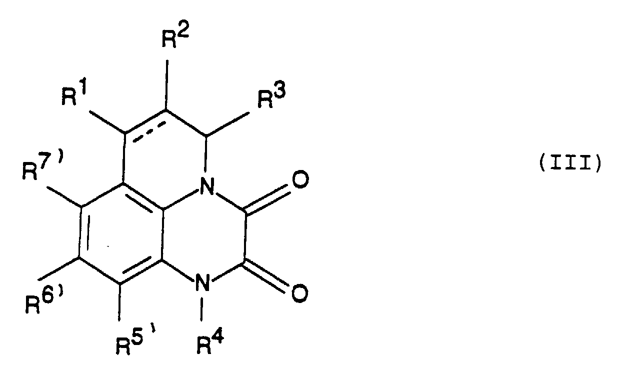 dans laquelle
R¹ à R⁴ ont les significations données ci-dessus et R^{5'}, R^{6'} ou R^{7'} représentent un atome d'hydrogène ou un groupe électrophile et, si on le souhaite, on saponifie ensuite un groupe ester, ou on estérifie ou amidifie un groupe acide, ou on réduit un groupe nitro en groupe amino, ou on alkyle ou acyle en groupe amino, ou on échange un groupe amino contre un atome d'halogène ou un groupe cyano, ou un introduit ou substitue de façon nucléophile un groupe nitro ou un atome d'halogène, ou on sépare les isomères ou on forme des sels.
